# EUROPEAN PATENT APPLICATION

(11) **EP 2 143 719 A1**
(43) Date of publication of application: **13.01.2010**
(21) Application number: 08739193.4
(22) Date of filing: 28.03.2008
(51) Int. Cl.: C07D 309/32, A23L 1/30, A61K 31/351, A61K 35/74, A61P 1/16, A61P 3/04, A61P 3/06, A61P 3/10, A61P 9/10, A61P 9/12, A61P 11/16, A61P 15/08, A61P 19/02, A61P 19/06, A61P 19/08, A61P 25/02

(54) **PHARMACEUTICAL COMPOSITION, FOOD OR BEVERAGE CAPABLE OF ENHANCING SYMPATHETIC NERVE ACTIVITY**

(30) Priority: 30.03.2007 JP 2007092244
(71) Applicant: Suntory Holdings Limited, Kita-ku, Osaka-shi Osaka 530-8203 (JP)
(72) Inventor: BEPPU, Yoshinori, Mishima-gun Osaka 618-8503 (JP); TSURUOKA, Nobuo, Mishima-gun Osaka 618-8503 (JP); ROGI, Tomohiro, Mishima-gun Osaka 618-8503 (JP); KOMURA, Hajime, Mishima-gun Osaka 618-8503 (JP); NAGAI, Katsuya, Minoh-shi Osaka 562-0044 (JP)
(74) Representative: Vossius & Partner
(86) International application number: PCT/JP2008/056075
(87) International publication number: WO 2008/120712

(57) **Abstract**

It is intended to provide a safe composition for preventing or treating obesity or related complications which has an autonomic nervous control effect, in particular, an effect of enhancing sympathetic nervous activity and an effect of promoting energy metabolism. Thus, a medicinal composition, a food or a drink containing 2,3-dihydro-3,5-dihydroxy-6-methyl-4H-pyran-4-one and having an autonomic nervous control effect and an effect of promoting energy metabolism is provided.

## Description

### TECHNICAL FIELD

The present invention relates to a medicinal composition, a food or a drink which contains 2,3-dihydro-3,5-dihydroxy-6-methyl-4H-pyran-4-one and has an autonomic nervous control effect. Further, it relates to the medicinal composition, food or drink as described above which has an effect of enhancing sympathetic nervous activity and an effect of promoting energy metabolism and is to be used for treating a disease or condition relating to the autonomic nervous activity or energy metabolism, in particular, obesity and/or related complications.

### BACKGROUND ART

To maintain the homeostasis, a living body has the autonomic nervous system, the endocrine system and the immune system. Among these systems, the autonomic nervous system is relatively free from the cerebral control and acts automatically without being consciously controlled, which is the origin of the name. It mainly controls visceral functions. The autonomic nervous system comprises the sympathetic nervous system and the parasympathetic nervous system and is controlled based on a balance between these two systems. When the body is in action, the sympathetic nervous activity become predominant and the whole body is in a state of tension. On the contrary, when the parasympathetic nervous activity become predominant, the tension is loosened and the body is relaxed (Non-Patent Document 1). Sympathetic hyperactivity causes pupil dilation, tachycardia, a rise in blood pressure, substance metabolism, hyperglycemia and so on. Parasympathetic hyperactivity causes activation of the digestion and absorption system, secretion of sweat and saliva and so on. It is also known that the autonomic nervous activity relate to control of the white adipose tissue and the brown adipose tissue functions. Namely, the white adipose tissue function is controlled by the sympathetic nerve and hormones in the blood, while the brown adipose tissue function is controlled mainly by the sympathetic nerve (Non-Patent Document 2).

On the other hand, obesity has become a serious health problem not only in developed countries but also in developing countries, so that antiobesity measures are required worldwide. In people having BMI (body mass index; a standard defining obesity) of 25 or higher, prevalences of various diseases such as hypertension, hyperglycemia and hyperglyceridemia show linear increases with an increase in BMI (Non-Patent Document 3). Also, it has been clarified that many pathological conditions induced by obesity relate not only to a degree of obesity but also to visceral fat accumulation.

Although antiobesity drugs have been developed and used around the world under these circumstances, it is also known that these drugs exert side effects. As a typical example thereof, sibutramine (Meridia manufactured by Knoll, etc.) has a possible risk of inducing an increase in heart rate or a rise in blood pressure, which makes it difficult to administer this drug to patients with symptoms frequently occurring in obese persons such as hypertension, cardiac angina, congestive heart failure or cerebral stroke. Orlistat (Xenical manufactured by Roche) sometimes shows serious side effects of diarrhea and steatorrhea.

In Japan, as antiobesity drugs, mazindol (Sanorex)(Non-Patent Document 4), which is reported as having an antiobesity effect via the activation of the brown adipose tissue, and *Farng Feng Tong San (bohutusyo-san)* have been mainly employed. *Farng Feng Tong San* is composed of 18 kinds of crude drugs including ephedra herb which contains 1-ephedrine and d-pseudophedrine and enhances the release of noradrenaline from the sympathetic nervous ending to thereby activate β-adrenaline receptor in the brown adipose tissue (Non-Patent Document 5). It is reported that these drugs exert side effects too. That is, it has been confirmed that mazindol causes thirst, constipation, nausea and sleep disorders while *Farng Feng Tong San* causes hepatic disorders.

Paying attention to therapeutic effects of lactic acid bacteria, in particular, there have been disclosed an antiobesity effect of a fermented product obtained by fermenting carrot root by using lactic acid bacteria (Patent Document 1), an effect of preventing visceral fat accumulation achieved by a lactic acid bacterium cultured product (Patent Document 2) and so on. However, no specific action on the white adipose tissue and the brown adipose tissue has been verified so far. Paying attention to health improvement using the autonomic nervous control effect of lactic acid bacteria, there has been merely reported an effect of alleviating constipation (Patent Document 3) but no effect on obesity has been disclosed so far.

On the other hand, concerning 2,3-dihydro-3,5-dihydroxy-6-methyl-4H-pyran-4-one which is contained in various foods and can be synthesized by a plurality of known methods, there have been known an inhibitory effect of tyrosinase, an inhibitory effect of melanogenesis (Patent Document 4) and an antioxidant effect (Patent Document 5) alone.
Patent Document 1: JP-A-2004-201598
Patent Document 2: JP-A-2004-99539
Patent Document 3: JP-A-2004-155727
Patent Document 4: JP-A-2006-28077
Patent Document 5: JP-A-H04-1291 (JP No. 2843639)
Non-Patent Document 1: Toshio Hagihara, et al., Iwanami Koza, Gendai Igaku no Kiso, vol.4, Seitai no Chousetsu Shisutemu, 1999
Non-Patent Document 2: Nippon Rinsho, vol. 6, extra ed.6, 288-294, 2003
Non-Patent Document 3: Himan Kenkyu 6:4-17, 2000
Non-Patent Document 4: Yoshida T., et al.: Clin. Exp. Pharmacol. Physiol. 23:476-482, 1996
Non-Patent Document 5: Nippon Rinsho, vol.61, extra. ed.6, 649-654, 2003

### DISCLOSURE OF THE INVENTION

### PROBLEMS THAT THE INVENTION IS TO SOLVE

There are a large number of autonomic nervous disorders, and of health disorders caused thereby. However, it has been a common practice to separately treat these disorders, and no attempt has been made so far to control the autonomic nervous activity per se that are the fundamental cause for these disorders. Although each symptom can be temporarily alleviated by such a symptomatic treatment, there still remains a high risk of recurrence or of the onset of another symptom caused by autonomic nervous disorders. Thus, there have been required drugs, foods and drinks whereby the autonomic nervous activity per se, i.e., the fundamental cause can be controlled.

Moreover, preventives and remedies for obesity and related complications that have been widely used hitherto exhibit side effects. Thus, it is difficult to establish the desired antiobesity effects, minimal side effects and high safety at the same time. Furthermore, a process for synthesizing a drug involves additional step(s) and a thus synthesized drug should be processed into a form assuring easy intake thereof such as tablets, which brings about another problem; high cost. Under these circumstances, it has been required to utilize the antiobesity effect of a safe food material or a food-origin component that has been taken by human beings for a long time and it has been also strongly required to develop an active substance which can be obtained from a natural material occurring in nature via a simple process.

No remedy for obesity based on the promotion of energy consumption has been developed so far (Nippon Rinsho, vol.61, extra ed.6, 686-691, 2003). Since the relationship between enlargement in adipose tissue and insulin resistance has been clarified, it is expected that an energy consumption promoter ensuring a qualitative change of enlarged adipose cells into small-sized cells having normal functions would be usable as an ideal remedy for obesity. When an antiobesity effect is to be achieved by enhancing the sympathetic nervous activity of adipose tissue from the above-described point of view, it is anticipated that blood pressure would be raised. Thus, this phenomenon should be avoided. Accordingly, it is ideal, if possible, that such an antiobesity effect based on the promotion of energy consumption having the characteristics as discussed above is provided with the use of a safe food material or a food-origin component that has been taken by human beings for a long time.

An object of the present invention is to provide a safe composition for preventing or treating obesity and/or related complications which have an autonomic nervous control effect; in particular, an effect of enhancing sympathetic nervous activity and, moreover, an effect of promoting energy metabolism.

### MEANS FOR SOLVING THE PROBLEMS

It has been confirmed that cells of a specific lactic acid bacterium can reduce sympathetic nervous activity of the kidney and enhance parasympathetic nervous activity of the stomach (Tanida M, et al., Neuroscience Letters 389:109-114, 2005).

To achieve the object as described above, the present inventors conducted intensive studies and isolated 2,3-dihydro-3,5-dihydroxy-6-methyl-4H-pyran-4-one from a culture supernatant of a lactic acid bacterium with the use of the direct effects on the rat autonomic nervous activity as an indication. Then, they have found that this compound has an effect of rather lowering blood pressure, though it has an effect of enhancing sympathetic nervous activity of the kidney and that it has another effect of enhancing sympathetic nervous activity of the white adipose tissue and the brown adipose tissue. Moreover, they have found that this compound has still another effect of promoting energy metabolism, thus completing the present invention.

Accordingly, the present invention relates to a medicinal composition, a food or a drink which contains a clinically effective amount of 2,3-dihydro-3,5-dihydroxy-6-methyl-4H-pyran-4-one and a pharmaceutically or nutritionally acceptable additive, and has an autonomic nervous control effect.
The invention further relates to the medicinal composition, food or drink as described above wherein the autonomic nervous control effect is an effect of enhancing sympathetic nervous activity.
The invention further relates to a medicinal composition, a food or a drink, which has an effect of promoting energy metabolism.
The invention further relates to the medicinal composition, food or drink as described above which is to be used for treating a disease or condition relating to autonomic nervous activity or energy metabolism.
The invention further relates to the medicinal composition, food or drink as described above wherein the disease or condition relating to the autonomic nervous activity or energy metabolism is obesity and/or related complications (for example, diabetes/impaired glucose tolerance, hyperlipemia, hypertension, hyperuricemia/gout, coronary artery diseases (heart infarction, angina pectoris, etc.), brain infarction (brain thrombosis, transient ischemic attack, etc.), sleep apnea syndrome, fatty liver, orthopedic diseases (arthritis deformans, lumbar disease, etc.), menstruation disorder and so on).
The invention further relates to the medicinal composition, food or drink as described above which contains a microorganism capable of producing 2,3-dihydro-3,5-dihydroxy-6-methyl-4H-pyran-4-one or a processed product thereof.
The invention further relates to the medicinal composition, food or drink as described above which contains a culture supernatant of a microorganism capable of producing 2,3-dihydro-3,5-dihydroxy-6-methyl-4H-pyran-4-one or a processed product thereof.
The invention further relates to the medicinal composition, food or drink as described above wherein the microorganism is a lactic acid bacterium.
The invention further relates to the medicinal composition, food or drink as described above wherein the lactic acid bacterium is one belonging to the genus *Streptococcus, Lactobacillus, Leuconostoc, Pediococcus, Bifidobacterium, Tetragenococcus, Weissella, Enterococcus, Melisscoccus, Lactococcus, Carnobacterium, Vagococcus, Atopobium, Lactosphaera, Oenococcus, Abiotrophia, Paralactobacillus, Granulicatella, Atopobactor, Alkalibacterium* or *Olsenella.*
The invention further relates to the medicinal composition, food or drink as described above wherein the microorganism belongs to *Lactobacillus plantarum* (preferably *Lactobacillus plantarum* SAM 2446 strain (FERM ABP-10438)) or *Lactobacillus brevis* (preferably *Lactobacillus brevis* SAM 2447 strain (FERM ABP-10439)).
The invention further relates to a method of producing the medicinal composition, food or drink as described above which comprises the step of culturing a microorganism capable of producing 2,3-dihydro-3,5-dihydroxy-6-methyl-4H-pyran-4-one in a medium.

The invention further relates to the medicinal composition, food or drink as described above wherein the autonomic nervous control effect is one or more effects (preferably all effects) selected from the group consisting of an effect of enhancing sympathetic nervous activity of the kidney, an effect of enhancing sympathetic nervous activity of the adrenal gland, an effect of reducing vagal (parasympathetic) nervous activity of the stomach, an effect of enhancing sympathetic nervous activity of the white adipose tissue and an effect of enhancing sympathetic nervous activity of the brown adipose tissue.
The invention further relates to the medicinal composition, food or drink as described above wherein autonomic nervous control effect is not accompanied by a rise in blood pressure. The invention further relates to the medicinal composition, food or drink as described above which has an effect of promoting energy metabolism and/or an effect of promoting fat burning.
The invention further relates to the medicinal composition as described above which is to be orally administered.
The invention further relates to an autonomic modulator which contains 2,3-dihydro-3,5-dihydroxy-6-methyl-4H-pyran-4-one as the active ingredient.
The invention further relates to use of 2,3-dihydro-3,5-dihydroxy-6-methyl-4H-pyran-4-one in producing a medicinal composition, a food or a drink for treating a disease or condition relating to autonomic nervous activity.
The invention further relates to a method of treating a disease relating to autonomic nervous activity which comprises administering 2,3-dihydro-3,5-dihydroxy-6-methyl-4H-pyran-4-one.
The invention further relates to an energy metabolism controlling agent which contains 2,3-dihydro-3,5-dihydroxy-6-methyl-4H-pyran-4-one as the active ingredient.
The invention further relates to use of 2,3-dihydro-3,5-dihydroxy-6-methyl-4H-pyran-4-one in producing a medicinal composition, a food or a drink for treating a disease or condition relating to energy metabolism.
The invention further relates to an autonomic nervous control method which comprises administering 2,3-dihydro-3,5-dihydroxy-6-methyl-4H-pyran-4-one.
The invention further relates to an energy metabolism controlling method which comprises administering 2,3-dihydro-3,5-dihydroxy-6-methyl-4H-pyran-4-one.

2,3-Dihydro-3,5-dihydroxy-6-methyl-4H-pyran-4-one (hereinafter sometimes referred to simply as "DDMP") to be used in the present invention is represented by the following formula.

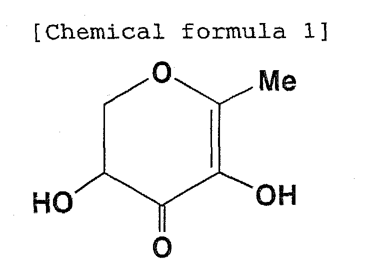

This compound is obtained in, for example, Example 1 as will be described hereinafter. A medicinal composition, a food or a drink (hereinafter sometimes referred to simply as "composition") containing DDMP has an autonomic nervous control effect; in particular, an effect of enhancing sympathetic nervous activity. Further, it has an effect of promoting energy metabolism, which makes it particularly useful in treating obesity and/or related complications.

It is known that DDMP, which is a compound analogous to maltol known as an aroma component, is contained in various foods and so on. For example, it is contained in various kinds of foods and so on, such as propolis, oak barrels, maguey, wine, brandy, milk, cookies, birch sap, popcorn, strawberry jam, blackstrap molasses, brown sugar and so on. DDMP increases in the course of aging of wine (Journal International des Sciences de la Vigne et du Vin 32:99-110, 1998) and brandy (Priklanda Biokhmiyai Mikrobiologiya 15:902-908, 1979). On the other hand, it is formed by a Maillard reaction in an oak barrel (Cutzach Izabelle et al.: Journal of Agricultural and Food Chemistry 45:2217-2224, 1997), maguey (Lopez, M.G., et al.: Frontiers of Flavour Science:523-526, 2000) and milk (Pischetsrieder, Monica et al.: Food Research and Technology, 208:172-177, 1999).

Therefore, DDMP and the DDMP-containing composition as described above can be obtained from foods, plants, etc. containing DDMP or materials in which DDMP has been formed by aging, heating, a Maillard reaction or the like. In forming DDMP, an optional procedure well known to a person skilled in the art may be conducted so as to increase the yield. To efficiently form the target compound by the Maillard reaction, for example, it is a common practice to add ascorbic acid or another substance capable of participating in the Maillard reaction.

As will be described hereinafter in Examples, the present inventors have also found that DDMP is contained in a culture supernatant of a lactic acid bacterium (*Lactobacillus plantarum* SAM 2446 strain (FERM ABP-10438)).

To separate DDMP from the above-described material, culture supernatant, etc., the starting material may be fractionated as such. Alternatively, it may be concentrated by freeze drying, liquid-liquid partition using an organic solvent or the like prior to the fractionation. The subsequent fractionation procedure may be carried out by, if desired, conducting of crude fractionation by ultrafiltration or the like followed by a treatment with the use of a reversed phase column, etc. The liquid-liquid partition, ultrafiltration, reversed phase column treatment, etc. as described above can be conducted by methods commonly employed by a person skilled in the art.

It is also possible to obtain DDMP by synthesis. Examples of the method of synthesizing DDMP include a method which comprises heating glucose with β-alanine (Nishibori S., et al.: Journal of Agricultural and Food Chemistry 41:2374-2377, 1993), a method which comprises heating sucrose with wine yeast (Mishiev, P.J., et al.: Prikladnyaba Biokhimiya i Mikrobiologiya 17:307-311, 1981), a method which comprises heating glucose with piperidine (Chen Yongkuan et al.: Huaxue Yanjiu Yu Yingyong 15:45-48, 2003), a method which comprises using glucose, piperidine and thioglycolic acid (Isabelle Cutzach et al.: J. Agric. Food Chem. 47:1663-67, 1999), a method which comprises heating a saccharide composition during hydrolysis of starch with L-arginine and glutamic acid, or a saccharide composition during hydrolysis of starch with glutamic acid (Song Wendong et al.: Fenix Huaxue 29:620, 2001) and so on, though the synthesis method is not particularly restricted.

The formation of DDMP by such a method can be confirmed by, for example, MS spectrometry or NMR spectrometry.

DDMP contained in the composition according to the present invention may be used in the form of a pharmaceutically acceptable salt such as hydrochloride or in the form of a salt (a prodrug) that is converted into DDMP in vivo. Also, use can be made of a D-compound, an L-compound or a racemate.

It has been reported that DDMP, which is an approved food additive perfume having a high safety, has an inhibitory effect of melanogenesis (Patent Document 4) and an antioxidant effect (Patent Document 5). However, it has never been reported so far that DDMP has an effect on the autonomic nerve, an effect on the sympathetic nerve, an effect of promoting energy metabolism, or an effect targeting the treatment of obesity and/or related complications.

The present invention also provides the medicinal composition, food or drink as described above which contains a microorganism capable of producing DDMP or a processed product of the same, or a culture supernatant of this microorganism or a processed product of the same.

The expression "capable of producing DDMP" as used herein means capable of producing DDMP when cultured under effective conditions. Such effective conditions can be appropriately determined by a person skilled in the art. The capability of producing DDMP of a microorganism can be confirmed by analyzing the microorganism (including a microorganism per se, dried cells of a microorganism, a culture medium of a microorganism, an extract of a microorganism and so on) by using a commonly employed procedure such as LC, MS or NMR to examine the presence of DDMP, etc. A microorganism capable of producing DDMP in the case of appropriately controlling the culture conditions (the culture composition, culture temperature, culture pH, culture density, etc.) also falls within the scope of the microorganism capable of producing DDMP.

The microorganism as described above may be collected from a natural source. Moreover, a variant and/or a recombinant designed as being capable of producing DDMP are also included in the scope of the present invention. A variant and/or a recombinant designed as showing an elevated DDMP productivity compared with a wild strain when cultured in a culture medium of the same composition also fall within the scope.

The microorganism capable of producing DDMP may be, for example, a lactic acid bacterium, a yeast, a hay bacillus or the like. Examples of the lactic acid bacterium include microorganisms belonging to the genus *Streptococcus, Lactobacillus, Leuconostoc, Pediococcus, Bifidobacterium, Tetragenococcus, Weissella, Enterococcus, Melisscoccus, Lactococcus, Carnobacterium, Vagococcus, Atopobium, Lactosphaera, Oenococcus, Abiotrophia, Paralactobacillus, Granulicatella, Atopobactor, Alkalibacterium* or *Olsenella*; examples of the yeast include microorganisms belonging to the genus *Candida* or *Saccharomyces*; and examples of the hay bacillus include microorganisms belonging to *Bacillus subtilis.*

Particularly preferable microorganisms are *Lactobacillus plantarum* (more specifically *Lactobacillus plantarum* SAM 2446 strain (FERM ABP-10438)) and *Lactobacillus brevis* (more specifically *Lactobacillus brevis* SAM 2447 strain (FERM ABP-10439)).

The present invention further provides a method of producing the medicinal composition, food or drink as described above which comprises the step of culturing the above-described microorganism capable of producing DDMP in a medium. The microorganism can be cultured by inoculation of an appropriate medium with the microorganism and employing of a culture procedure that is well known to a person skilled in the art, depending on the kind of the microorganism.

Next, the culture in the case where the microorganism is a lactic acid bacterium will be briefly described by way of example. As the medium, use can be made of, for example, an agar medium and/or a liquid medium. To the medium, a carbon source and a nitrogen source well known to a person skilled in the art are optionally added, each at a desired concentration. If necessary, trace nutrients such as an inorganic ion and a vitamin may be further added. More conveniently, use can be made of a marketed medium such as MRS medium to which additives may be optionally added. After preparation of the medium, it is adjusted to pH 6.0 to 7.0 with the use of an appropriate acid or base and then sterilized using an autoclave or the like.

Subsequently, the medium is inoculated with the lactic acid bacterium. Then, the microorganism can be proliferated in the medium by shaken culture, static culture, industrial-scaled culture in a tank, solid culture in a solid medium such as an agar medium, etc. while the culture temperature is controlled to 10°C to 45°C usually for 1 to 2 days. The culture conditions vary depending on the microorganism employed. In the case of using *Lactobacillus plantarum,* for example, static culture can be conducted in MRS medium of around pH 6.5 at around 37°C for 1 day. The microorganism thus cultured is centrifuged if desired, and then filtered if necessary. Thus, a culture supernatant can be obtained.

As will be shown in Examples hereinafter, DDMP has an effect of enhancing the rat kidney sympathetic nervous activity without causing a rise in blood pressure when transduodenally administered to rats. Further, it has an effect of reducing vagal (parasympathetic) nervous activity of the stomach, an effect of enhancing sympathetic nervous activity of the interscapular brown adipose tissue and an effect of enhancing sympathetic nervous activity of the epididymal white adipose tissue in rats. That is to say, DDMP is intestinal canal-inhibitive, enhances sympathetic nervous activity (the kidney and the adrenal gland), reduces vagal (parasympathetic) nervous activity of the stomach and further enhances sympathetic nervous activity of the white adipose tissue and the brown adipose tissue without causing a rise in blood pressure.

As will be shown in Examples hereinafter, DDMP caused an increase in the rat oxygen consumption when forcibly orally administered into the stomach of rats, which indicates that DDMP has an effect of promoting energy metabolism.

As methods for evaluating autonomic nervous activity, there can be enumerated biophysical measurement methods using, for example, an electrocardiograph, a sphygmomanometer, a galvanic skin reflex meter or a pupil meter and biochemical methods of measuring blood catecholamine concentration, etc. Although analysis on heart rate change using an electrocardiograph is a commonly employed method, the present inventors evaluated the effects of DDMP on autonomic nervous activity by a method using rats, as will be shown in Examples hereinafter, whereby the effects of the administered substance on the autonomic nervous activity can be directly evaluated. This method is **characterized in that** the effects of the administered substance on the autonomic nervous activity controlling individual organs, tissues and so on can be separately examined. Moreover, this method has a merit that autonomic nervous control functions of different substances can be successively evaluated so long as the test animal survives.

The following table shows organs, hormones and vital actions seemingly participating in autonomic nervous activity, possible effects of the autonomic nerve thereon, and examples of the expected effects.

**[Table 1]**

| | | Sympathomimetic | Parasympathomimetic |
|---|---|---|---|
| Pancreas | Insulin | Decrease | Increase |
| | Glucagon | Increase | Decrease |
| Adrenal gland | Blood glucose | Increase | Decrease |
| | Blood pressure | Increase | Decrease |
| Liver | Glycogen | Decomposition | Synthesis |
| | Sugar | Gluconeogenesis | Glycolysis |
| | | →Sugar synthesis | enhancement |
| | | | →Sugar use promotion |
| Kidney | Blood pressure | Increase | Decrease |
| Stomach | Digestion/ | Decrease | Promotion |
| | absorption | →Loss of | →Appetite |
| | | appetite | enhancement |
| | | (antiobesity) | |
| Skin | Blood flow | Decrease | Increase |
| rate | | | →Mozsturization en- |
| | | | hancement (cosmetic) |
| Immunity | | Decrease | Increase |
| | | | →NK activity rise |
| Pupil | | Enlargement | Contraction |
| Heart rate | | Increase | Decrease |
| Cardiovascular system | | Enhancement | Regulation |
| | | (facial pallor) | (facial flush) |
| Sweat and saliva | | Secretion | Secretion enhancement |
| | | regulation | |

As the above table clearly shows, the autonomic nervous activity closely affect various controlling mechanisms *in vivo,* for example, blood sugar control, blood pressure control, hormone secretion control, sugar metabolism control, gastric juice secretion control, blood flow rate control, body temperature control and so on. When the sympathetic nervous activity are enhanced, for example, there arise fat decomposition in the white adipose tissue and heat production enhancement in the brown adipose tissue (Non-Patent Document 2), sugar synthesis due to gluconeogenesis in the liver, and a decrease in the digestion/absorption in the stomach. When the parasympathetic nervous activity are enhanced, there arise promotion of sugar use due to glycolysis enhancement in the liver, appetite enhancement in the stomach, moisturization enhancement due to an increase in blood flow rate in the skin and immunopotentiation due to a rise in NK activity in the immunity.

When particular attention is paid to antiobesity measures, visceral fat, among the fats relating to obesity, has a higher metabolic activity than subcutaneous fat. Therefore, it is known that visceral fat is quickly synthesized at hyperalimentation and vigorously synthesized and decomposed during exercise or in cases of malnutrition. Accordingly, it is considered that fat synthesis and decomposition are both vigorously conducted in an individual with visceral fat accumulation and the resulting free fatty acids that are the metabolites are released in a large amount. In addition, these free fatty acids released from the visceral fat would flow directly into the liver via the portal vein. The excessive inflow of the free fatty acids regulates the insulin clearance in the liver and potentiates gluconeogenesis, thereby elevating blood sugar level. As a result, peripheral insulin resistance is induced. It is considered that obesity results in impaired glucose tolerance through this mechanism (Matsuzawa Y.: Diabetes Metab. Rev. 13:3-13, 1997). On the other hand, the onset mechanism from obesity to hyperlipemia seemingly proceeds as follows: namely, a mechanism mediated by an increase in the excessive inflow of the free fatty acids from the visceral fat into the liver followed by an increase in fat synthesis substrates and enhancement of expression of lipid synthesis-relating protein genes; or a mechanism mediated by the promotion of exogenous cholesterol absorption due to insulin resistance (Kuriyama H. et al.: Hepatology 27:557-562, 1998).

When the cross-talk of adipose tissue function and autonomic nervous activity is taken into consideration from the viewpoint of factors causative of obesity, the adipose tissue includes the white adipose tissue serving as a storage of fat energy and the brown adipose tissue oxidizing and decomposing fat and releasing energy from the body as heat. The white adipose tissue is widely distributed all over the body, mainly around visceral tissues and subcutaneous tissues. It is a specialized organ for storing excessive energy after food intake and re-supplying it as fatty acids and glycerol to the whole body. It is also reported that a decrease in the fat decomposition by the white adipose tissue and a decrease in the activity of the brown adipose tissue result in obesity (Nicholls, et al.: Physiol. Rev. 64:1-64, 1984).

The autonomic nervous activity participate in the control of the functions of the white adipose tissue and the brown adipose tissue. Namely, the function of the white adipose tissue is controlled by the sympathetic nervous and blood hormones, while the function of the brown adipose tissue is controlled mainly by the sympathetic nervous (Non-Patent Document 2). When the sympathetic nervous activity of the white adipose tissue is enhanced, fat decomposition is promoted. When the sympathetic nervous activity of the brown adipose tissue is enhanced, fat oxidation and decomposition are promoted and energy is released from the body as heat.

The term "autonomic nervous control effect" as used herein includes an effect of enhancing sympathetic nervous activity, an effect of reducing sympathetic nervous activity enhancement, an effect of reducing sympathetic nervous activity, an effect of enhancing parasympathetic nervous activity, an effect of reducing parasympathetic nervous activity enhancement and an effect of reducing parasympathetic nervous activity. It preferably means an effect selected from among an effect of enhancing sympathetic nervous activity, an effect of reducing parasympathetic nervous activity enhancement and an effect of reducing parasympathetic nervous activity. More specifically, it means an effect selected from among an effect of enhancing the sympathetic nervous activity in the kidney, an effect of enhancing the sympathetic nervous activity in the adrenal gland, an effect of reducing vagal (parasympathetic) nervous activity of the stomach, an effect of enhancing sympathetic nervous activity of the white adipose tissue and an effect of enhancing sympathetic nervous activity of the brown adipose tissue.

Because it contains DDMP, the composition according to the present invention is usable in treating a disease or condition relating to autonomic nervous activity; for example, symptoms frequently occurring due to autonomic nervous disorders such as shortness of breath, palpitation, shoulder stiffness, headache, vertigo, anxiety, loss of appetite, lassitude, sleep disorder, etc. It is particularly useful in treating a disease or condition which can be treated by enhancing sympathetic nervous activity; for example, obesity. Also, the composition according to the present invention reduces the vagal (parasympathetic) nervous activity of the stomach so as to regulate increased appetite and enhances the sympathetic nervous activity of the white adipose tissue and the brown adipose tissue without causing a rise in blood pressure. Owing to these functions, the composition according to the present invention promotes fat decomposition in these adipose tissues and thus prevents fat accumulation, which makes it useful in treating obesity and/or related complications (for example, diabetes/impaired glucose tolerance, hyperlipemia, hypertension, hyperuricemia/gout, coronary artery diseases (heart infarction, angina pectoris, etc.), brain infarction (brain thrombosis, transient ischemic attack, etc.), sleep apnea syndrome, fatty liver, orthopedic diseases (arthritis deformans, lumbar disease, etc.), menstruation disorder and so on).

The composition according to the present invention enhances sympathetic nervous activity of the white adipose tissue so as to promote fat decomposition. Also, it enhances sympathetic nervous activity of the brown adipose tissue and promotes oxidation and decomposition of fat to thereby release energy from the body as heat, which makes it effective in enhancing energy metabolism. The enhancement of energy metabolism can be evaluated by, for example, measuring the oxygen consumption *in vivo*.

The expression "to treat a disease or condition" as used herein means to prevent worsening of the disease or condition, to relieve the disease or condition, and to prevent the disease or condition.

The composition according to the present invention containing DDMP contains DDMP in a clinically effective amount. The clinically effective amount means an amount clinically effective in controlling the autonomic nerve. Based on the results of Examples, as will be described hereinafter, it is determined that a sufficient effect can be obtained by administering 0.5 µg/kg or more of DDMP. Although there is no upper limit of the administration dose of DDMP, it is generally favorable that the DDMP dose does not exceed about 50 mg/kg from the viewpoint of the characteristic smell thereof, and in view of cost. To fully achieve the effect, it is desirable that the composition according to the present invention contains from 0.5 µg/kg to 50 mg/kg per dose (preferably from 1 µg/kg to 10 mg/kg and more preferably from 2 µg/kg to 5 mg/kg) of DDMP. More specifically, it is desirable that the composition according to the present invention contains from 30 µg to 3000 mg (preferably from 60 µg to 600 mg and more preferably from 120 µg to 300 mg) per dose of DDMP in the case of administering to an adult human. It is possible that the composition according to the present invention contains from 0.05 µg to 5 mg (preferably from 0.1 µg to 1 mg and more preferably from 0.2 µg to 0.5 mg) of DDMP per product weight (g). From another viewpoint, it is possible that the composition according to the present invention contains 25 µg or more (preferably 40 µg or more and more preferably 80 µg or more) of DDMP per product weight (g). In each case, the upper limit may be adjusted to, for example, 0.5 mg, 1 mg or 5 mg per product weight (g).

In the composition according to the present invention, use can be made of a material containing DDMP, a microorganism capable of producing DDMP, a culture supernatant of the microorganism, a culture containing the microorganism, etc. either as such or after isolation and purification via extraction, fractionation or the like, though the present invention is not particularly restricted thereto. It is also possible to use a processed product such as a concentrate or a dry powder obtained by treating such a material as described above by using a commonly employed procedure such as sterilization, vacuum concentration, freeze drying or the like. In the case of a concentrate which can be hardly powdered by drying, it may be mixed with an excipient commonly employed in the art such as dextrin, a high-molecular starch hydrolyzate or a high-molecular peptide and then powdered by drying. From the viewpoints of handling properties and storage properties, a powdered composition is preferred.

The composition according to the present invention can be processed into various forms such as foods and drinks (including foods, drinks, seasonings, alcoholic drinks, functional foods and so on) and medicinal compositions. For example, it is possible to provide a food, a drink or a medicine with the use of DDMP, a microorganism capable of producing DDMP, a culture supernatant of the microorganism, a processed product thereof and so on.

Examples of the foods and drinks provided by the present invention include various ones such as candies, troches, yogurts, ice creams, puddings, jellies, *mizu-yokan* (adzuki bean jelly), alcoholic drinks, coffee drinks, juices, fruit drinks, carbonated drinks, soft drinks, milk, milk serum drinks, lactic acid drinks and so on.

These foods and drinks can be prepared by conventional methods with the use of various additives if needed. More specifically, these foods and drinks can be produced by blending of appropriate materials commonly employed in foods such as glucose, fructose, sucrose, maltose, sorbitol, stevioside, rubusoside, corn syrup, lactose, citric acid, tartaric acid, malic acid, succinic acid, lactic acid, L-ascorbic acid, dl-a-tocopherol, sodium erythorbate, glycerol, propylene glycol, a glycerol fatty acid ester, a polyglycerol fatty acid ester, a sucrose fatty acid ester, a sorbitan fatty acid ester, a propylene glycol fatty acid ester, acacia, carrageenan, casein, gelatin, pectin, agar, vitamin Bs, nicotinamide, calcium panthothenate, amino acids, calcium salts, a colorant, a perfume, a preserver and so on.

The medicines provided by the present invention may be in various dosage forms that are prepared by conventional methods with the use of various additives if needed. For example, it is possible to provide medicines for oral administration such as tablets, capsules, granules, powder, syrups and extracts and medicines for parenteral administration such as ointments, eye ointments, lotions, creams, patches, suppositories, eye drops, nasal drops and injections.

These medicines may be produced by conventional methods with the use of various additives. Any additives commonly employed in the art can be used without specific restriction. Examples thereof include solid carriers such as starch, lactose, sucrose, mannitol, carboxymethylcellulose, corn starch and inorganic salts; liquid carriers such as distilled water, physiological saline, aqueous glucose solution and alcohols such as ethanol, propylene glycol and polyethylene glycol; and oily carriers such as various animal or vegetable oils, white vaseline, paraffin and waxes.

The composition according to the present invention contains DDMP as the active ingredient, optionally together with other active ingredient(s) commonly employed in medicinal compositions, foods and drinks.

For example, the composition according to the present invention may contain one member or a combination of two or more members selected from among a substance having an autonomic control effect (a sympathetic agents, a parasympathetic agents, etc.), a hypotensive substance, an antiobesity substance (a substance stimulating the appetite regulatory system, a substance acting on the satiety center, a substance participating in energy metabolism (for example, a β₃ receptor agents, a substance promoting energy consumption), etc.) that are well known to a person skilled in the art.

In the case of the composition according to the present invention containing a microorganism capable of producing DDMP, a processed product thereof, a culture supernatant thereof or a processed product thereof, it may contain, in particular, the above-described substances with microbial origin (a substance having an autonomic control effect, a hypotensive substance, a substance promoting energy metabolism, etc.).

In the medicinal composition, food or drink according to the present invention, indication may be made of a specific purpose of use (for example, controlling the autonomic nerve, enhancing the sympathetic nervous activity, regulating the parasympathetic nervous activity, preventing obesity, treating obesity, treating a complication of obesity, promoting energy metabolism, promoting fat burning or maintaining health) and/or a specific way of use (for example, dose, dosing frequency and dosing method).

### EFFECTS OF THE INVENTION

As discussed above in detail, the present invention provides a medicinal composition, a food or a drink for controlling autonomic nervous activity per se (in particular, enhancing the sympathetic nervous activity) that are the fundamental factors causative of a disease or condition relating to the autonomic nervous activity. In particular, it provides a medicinal composition, a food or a drink having an effect of enhancing the sympathetic nervous activity of the white adipose tissue and the brown adipose tissue without causing a rise in blood pressure. Further, it provides a medicinal composition, a food or a drink having an effect of promoting energy metabolism. Thus, it provides a medicinal composition, a food or a drink which is efficacious and highly safe in treating obesity and/or related complications with little side effect (no rise in blood pressure) and can be conveniently produced.

### BRIEF DESCRIPTION OF THE DRAWINGS

[Fig. 1] Fig. 1 presents an MS spectrum of 2,3-dihydro-3,5-dihydroxy-6-methyl-4H-pyran-4-one obtained in Example 1.
[Fig. 2] Fig. 2 presents NMR spectra ((a) showing a 13C-NMR spectrum, while (b) showing a 1H-NMR spectrum) of 2,3-dihydro-3,5-dihydroxy-6-methyl-4H-pyran-4-one obtained in Example 1.
[Fig. 3] Fig. 3 is a graph which shows the effect of enhancing the rat kidney sympathetic nervous activity of 2,3-dihydro-3,5-dihydroxy-6-methyl-4H-pyran-4-one obtained in Example 1.
[Fig. 4] Fig. 4 is a graph which shows that 2,3-dihydro-3,5-dihydroxy-6-methyl-4H-pyran-4-one obtained in Example 1 causes no rise in blood pressure.
[Fig. 5] Fig. 5 is a graph which shows the effect of reducing rat stomach vagal (parasympathetic) nervous activity of 2,3-dihydro-3,5-dihydroxy-6-methyl-4H-pyran-4-one obtained in Example 1.
[Fig. 6] Fig. 6 is a graph which shows the effect of enhancing sympathetic nervous activity of the rat interscapular brown adipose tissue of 2,3-dihydro-3,5-dihydroxy-6-methyl-4H-pyran-4-one obtained in Example 1.
[Fig. 7] Fig. 7 is a graph which shows the effect of enhancing the rat white adipose tissue sympathetic nervous activity of 2,3-dihydro-3,5-dihydroxy-6-methyl-4H-pyran-4-one obtained in Example 1.
[Fig. 8] Fig. 8 is a graph which shows the effect of promoting rat energy metabolism of 2,3-dihydro-3,5-dihydroxy-6-methyl-4H-pyran-4-one obtained in Example 1.

### EXAMPLES

Now, the present invention will be described in greater detail by reference to the following Examples, though the present invention is not restricted thereto.

In the present Examples, 2,3-dihydro-3,5-dihydroxy-6-methyl-4H-pyran-4-one was prepared by using a culture supernatant of *Lactobacillus plantarum* SAM 2446 strain (FERM ABP-10438) as the starting material and conducting a fractionation treatment. Then, the effects of 2,3-dihydro-3,5-dihydroxy-6-methyl-4H-pyran-4-one on rat autonomic nervous activity were examined.

In the present Examples, moreover, 2,3-dihydro-3,5-dihydroxy-6-methyl-4H-pyran-4-one was prepared by using glucose, piperidine and thioglycolic acid. Then, the effects of 2,3-dihydro-3,5-dihydroxy-6-methyl-4H-pyran-4-one on rat energy metabolism was examined.
In the present Examples, furthermore, medicines, foods and drinks were produced using DDMP.

### Example 1: Preparation of 2,3-dihydro-3,5-dihydroxy-6-methyl-4H-pyran-4-one (purification from lactic acid bacterium culture supernatant)

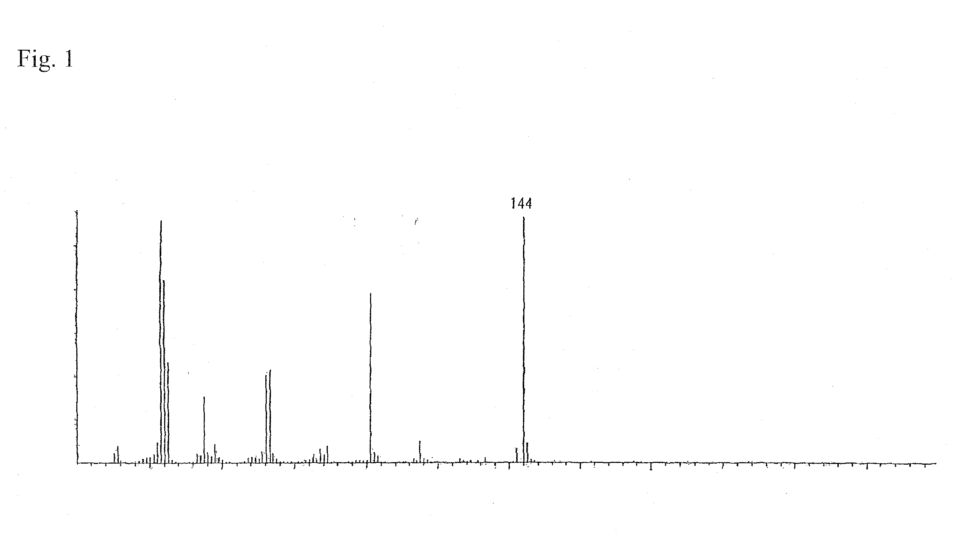

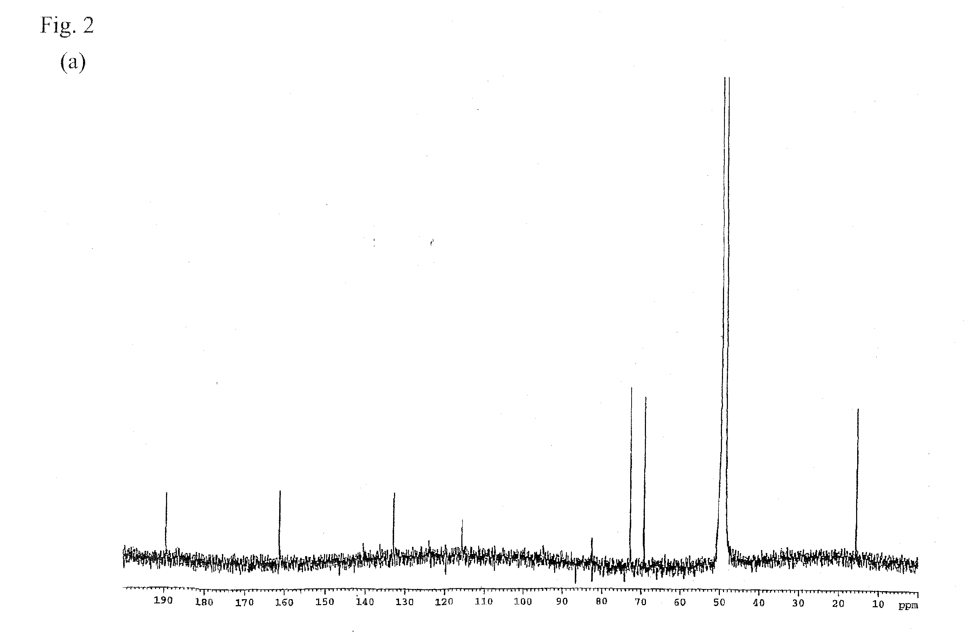

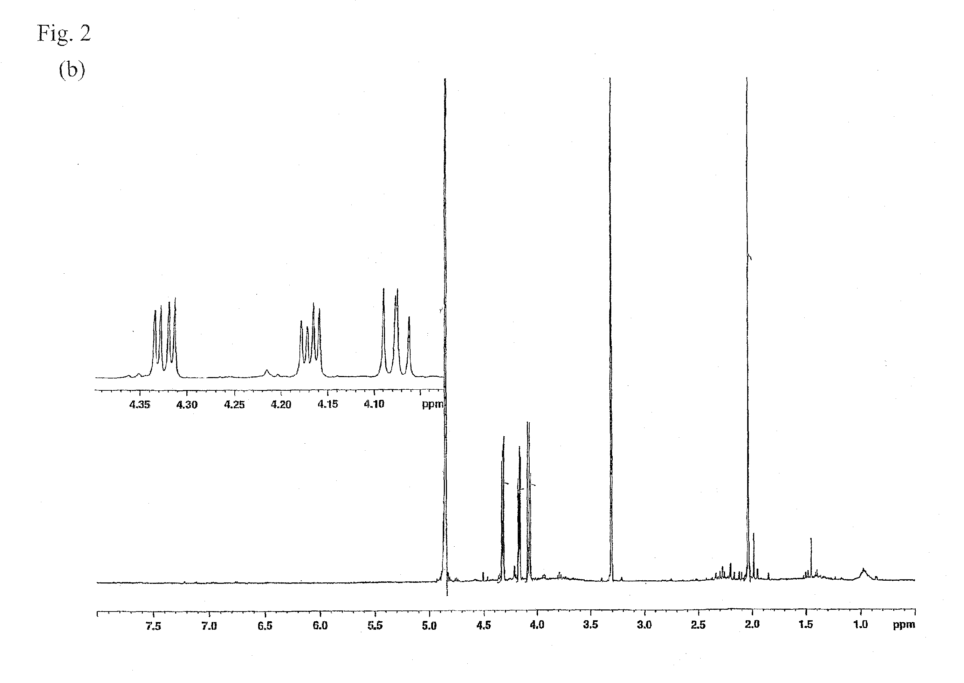

As the lactic acid bacterium culture supernatant, use was made of a freeze-dried culture supernatant obtained by culturing *Lactobacillus plantarum* SAM 2446 strain (FERM ABP-10438) in MRS medium. A sterilized MRS medium was inoculated with *Lactobacillus plantarum* SAM 2446 strain (FERM ABP-10438) which was then statically cultured therein at 37°C for 1 day. Next, the cells were removed by centrifugation to give a culture supernatant. By use of the culture supernatant thus obtained, a freeze dried product was prepared by a conventional method and employed as the starting material. First, the lactic acid bacterium culture supernatant was ultra-filtered using Ultrafiltration Membranes YM10 (manufactured by Millipore) to give a low molecular fraction passing through the membranes. Next, the obtained fraction was chromatographically fractionated through Develosil C30 UG-5 Column (manufactured by NOMURA CHEMICAL Co., Ltd.) with the use of the effect on the kidney sympathetic nervous activity observed in transduodenal administration as an indication, thereby giving 2,3-dihydro-3,5-dihydroxy-6-methyl-4H-pyran-4-one. Figs. 1 and 2 present MS and NMR data of this compound. In this method, the yield of 2,3-dihydro-3,5-dihydroxy-6-methyl-4H-pyran-4-one was 1.78 mg per 100 ml of the lactic acid bacterium culture supernatant.

### Example 2: Evaluation of the effects of 2,3-dihydro-3,5-dihydroxy-6-methyl-4H-pyran-4-one on rat kidney sympathetic nervous activity, blood pressure and stomach vagal (parasympathetic) nervous activity

In these experiments, use was made of male Wister rats weighing about 300 g which had been fed in a thermostatic chamber (24°C) with a light-dark cycle of 12-hour intervals (under illumination from 8:00 to 20:00) for 1 week or longer. A single rat was employed for each nervous experiment. The animals were fed with a feed (Oriental Yeast, MF) and water ad *libitum.* The autonomic nervous activity were examined by fasting the rats for 3 hours on the day of the experiment and then subjecting to an abdominal surgery under urethane-anesthesia at the intermediate point of the light period. Then, the effects of the transduodenal administration of 2,3-dihydro-3,5-dihydroxy-6-methyl-4H-pyran-4-one obtained in Example 1 on the kidney sympathetic nervous activity, stomach vagal (parasympathetic) nervous activity and blood pressure were measured in accordance with the method reported by T. Yamano et al., Neurosci. Lett. 313:78-82 (2001), A. Niijima et al., Autonom. Neurosci.: Basic & Clinical 97:99-102 (2002) and M. Tanida et al., Am. J. Physiol: Regulatory, Integrative and Comparative Physiology 288:R447-R455 (2005). Namely, the sympathetic nerve controlling the kidney and the parasympathetic nerve controlling the stomach were picked up using a silver electrode. A cannula was inserted into the left femoral artery and then the blood pressure was measured by use of a transducer. From the initiation of the surgery to the completion of the measurement, a tube was inserted into the respiratory tract for the management of the airway. The body temperature (rat rectal temperature) was maintained at 35±0.5°C with the use of a heater. The transduodenal administration was conducted by dissolving 2,3-dihydro-3,5-dihydroxy-6-methyl-4H-pyran-4-one obtained in Example 1 in physiological saline to give a concentration of 10 µg/2 ml, and administering 2 ml/animal of the obtained solution at a rate of 1 ml/min through a polyethylene tube inserted into the duodenum. Electrical nervous activity thus obtained were amplified with a condenser type differential amplifier, monitored with an oscilloscope and recorded on a magnetic tape. To separate the raw data from the background noise, all of the nervous activity were converted into standard pulses by use of a slicer and a window discriminator before analysis.
Discharge frequencies were indicated on a pen recorder by use of a rate meter at a reset time of 5 seconds. The data was analyzed based on the average discharge frequency per 5 seconds (pulse/5 sec) and expressed as a percentage by referring to the level before the administration as to 100%. A group with the administration of physiological saline was employed as a control.

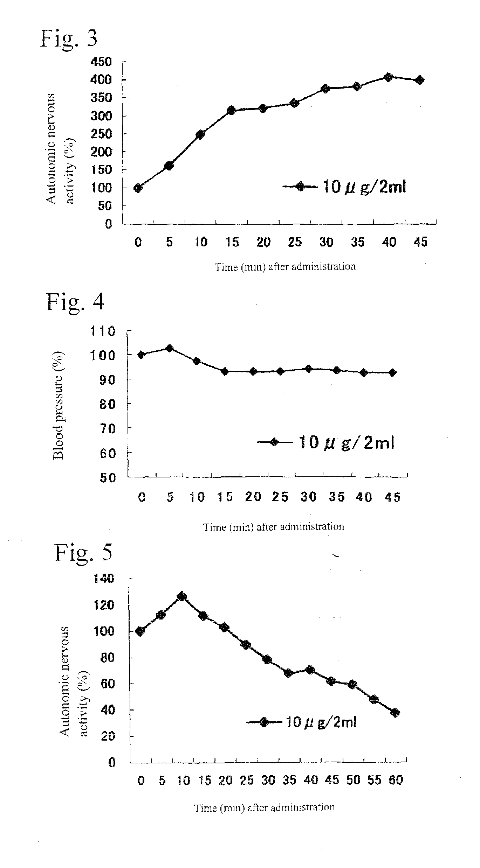

As a result, the control group showed no effect on all of the autonomic nervous activity and blood pressure. In the administration group, on the other hand, the kidney sympathetic nervous activity was enhanced (Fig. 3) while the blood pressure was not elevated (Fig. 4) and the stomach vagal (parasympathetic) nervous activity was reduced (Fig. 5).

### Example 3: Evaluation of the effects of 2,3-dihydro-3,5-dihydroxy-6-methyl-4H-pyran-4-one on rat interscapular brown adipose tissue sympathetic nervous activity and epididymal white adipose tissue sympathetic nervous activity

The procedure of Example 2 was followed and thus the effects of the transduodenal administration (10 µg) of 2,3-dihydro-3,5-dihydroxy-6-methyl-4H-pyran-4-one obtained in Example 1 on the rat epididymal white adipose tissue sympathetic nervous activity and the interscapular brown adipose tissue sympathetic nervous activity were examined in accordance with the methods reported in the documents cited in Example 2. A single rat was employed for each nervous experiment.

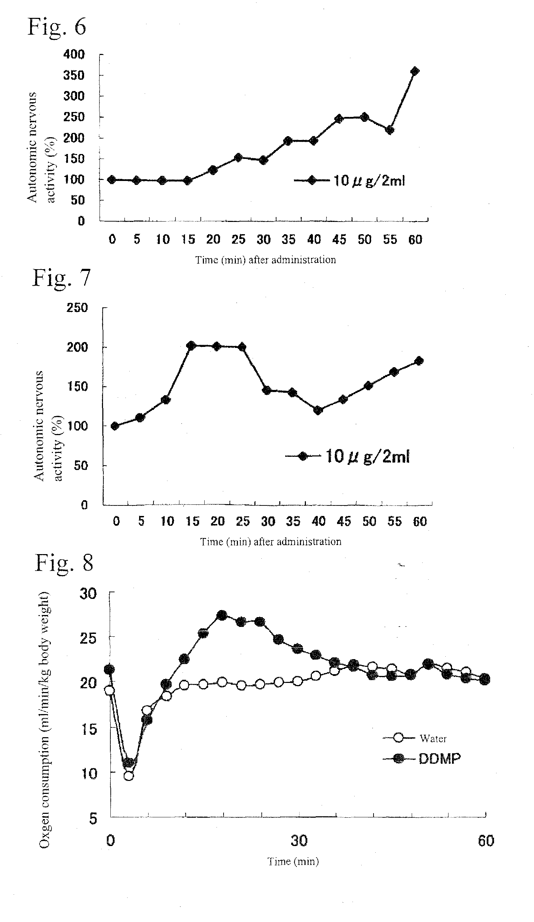

As a result, both of the interscapular brown adipose tissue sympathetic nervous activity (Fig. 6) and the epididymal white adipose tissue sympathetic nervous activity (Fig. 7) were enhanced.

### Example 4: Preparation of 2,3-dihydro-3,5-dihydroxy-6-methyl-4H-pyran-4-one (synthesis with the use of glucose, piperidine and thioglycolic acid)

2,3-Dihydro-3,5-dihydroxy-6-methyl-4H-pyran-4-one was prepared by use of glucose, piperidine and thioglycolic acid in accordance with Isabelle Cutzach et al.: J. Agric. Food Chem. 47:1663-67, 1999 (modified). Next the synthesis method reported in this document will be briefly described.

To a suspension of D-glucose (600 g, 3.33 mol) and triethylamine (192 mL, 3.33 mol), piperidine (329 mL, 3.33 mol) and acetic acid (192 mL, 3.33 mol) were added under stirring at room temperature. The obtained mixture was stirred under heating at 70°C for 1 hour. After cooling to room temperature by standing, the mixture was allowed to stand in a refrigerator overnight. The solid thus precipitated was washed with 4 L of a solvent mixture of ethanol-acetone (1:1) and collected by filtration. Then, it was heated to 35°C and dried under reduced pressure to give a piperidine derivative.

A solution of the obtained piperidine derivative (176 g, 0.711 mol) in ethanol (1.6 L) was stirred under heating at 75°C. A solution of thioglycolic acid (49.6 ml, 0.713 mol) in ethanol (135 mL) was dropped thereinto over 40 minutes and the mixture was heated to 75°C and stirred for additional 20 hours. After completion of the reaction, the mixture was concentrated under reduced pressure to give crude 2,3-dihydro-3,5-dihydroxy-6-methyl-4H-pyran-4-one. The crude 2,3-dihydro-3,5-dihydroxy-6-methyl-4H-pyran-4-one thus obtained was subjected to column chromatography successively with Daiso SP 120-40/60-ODS-B (manufactured by DAISO) and CHP20P (manufactured by MITSUBISHI CHEMICAL Co.). Thus a purified 2,3-dihydro-3,5-dihydroxy-6-methyl-4H-pyran-4-one preparation was obtained.

In the above synthesis method, the purified 2,3-dihydro-3,5-dihydroxy-6-methyl-4H-pyran-4-one preparation was obtained at a yield of 6.93 g of the purified 2,3-dihydro-3,5-dihydroxy-6-methyl-4H-pyran-4-one preparation per 600 g of glucose.

### Example 5: Effect of promoting energy metabolism of 2,3-dihydro-3,5-dihydroxy-6-methyl-4H-pyran-4-one

Male SD rats aged 7 weeks were preliminarily fed with a solid feed (CE-2: NIPPON CREA) and water ad *libitum* for 1 week for the habituation to the light-dark cycle of 12-hour intervals. The thus habituated animals were fasted for 16 hours before the administration of the samples and then divided into two groups. Then, 40 µg/8 ml/kg body weight of DDMP and 8 ml/kg body weight of distilled water for injection were forcibly orally administered into the stomach to the respective groups. Within 60 minutes following the administration, oxygen consumption was measured with a metabolic meter for small animals (MK-5000RQ2: manufactured by MUROMACHI KIKAI Co., Ltd.). A combination of a DDMP-administered rat with a water-administered rat was referred to as a single experimental lot and the averages of five combinations (i.e., a DDMP-administered group having 5 rats and a water-administered group having 5 rats) were calculated.

As a result, the DDMP-administered group showed an increase in oxygen consumption compared with the water-administered group. This difference was observed from 15 to 33 minutes after the administration (Fig. 8). Based on these results, it was confirmed that DDMP has an effect of promoting energy metabolism.

### Production Example 1: DDMP-containing medicine

### Tablet:

A DDMP-containing medicine (tablets) was produced by the following method.

2 g of DDMP obtained in Example 4, 296.7 g of lactose and 1.3 g of magnesium stearate were mixed together and tabletted with a single-shot tabletting machine to give tablets, each being 10 mm in diameter and 300 mg in weight.

### Granules:

2 g of DDMP obtained in Example 4 was added to 296.7 g of lactose and 1.3 g of magnesium stearate and the mixture was compressed, powdered, dressed and sieved to give granules of 20-50 mesh.

### Production Example 2: DDMP-containing food and drink

Various DDMP-containing foods and drinks of the following compositions were produced by conventional methods.

| Ice cream: | |
|---|---|
| (Component) | (Part by weight) |
| Fresh cream (fat: 45%) | 33.8 |
| Skim milk powder | 11.0 |
| Granulated sugar | 14.8 |
| Sugar-containing yolk | 0.3 |
| Vanilla essence | 0.1 |
| Water | 39.998 |
| DDMP | 0.002 |
| Total | 100.00 |

| Juice: | |
|---|---|
| (Component) | (Part by weight) |
| *Unshu* orange juice concentrate (frozen) | 5.0 |
| High-fructose corn syrup | 11.0 |
| Citric acid | 0.2 |
| L-Ascorbic acid | 0.02 |
| DDMP | 0.002 |
| Perfume | 0.2 |
| Colorant | 0.1 |
| Water | 83.478 |
| Total | 100.00 |

| Lactic acid drink: | |
|---|---|
| (Component) | (Part by weight) |
| Fermented milk (solid milk content: 21%) | 14.76 |
| High-fructose corn syrup | 13.31 |
| Pectin | 0.5 |
| Citric acid | 0.08 |
| Perfume | 0.15 |
| Water | 71.198 |
| DDMP | 0.002 |
| Total | 100.00 |

| Yogurt: | |
|---|---|
| (Component) | (Part by weight) |
| Fresh milk (fat: 3.4%) | 80.0 |
| Fresh cream (fat: 50%) | 8.0 |
| Skim milk powder | 1.5 |
| Water | 7.498 |
| Starter | 3.0 |
| DDMP | 0.002 |
| Total | 100.00 |

| Coffee drink: | |
|---|---|
| (Component) | (Part by weight) |
| Granulated sugar | 8.0 |
| Skim milk powder | 5.0 |
| Caramel | 0.2 |
| Coffee extract | 2.0 |
| Perfume | 0.1 |
| Polyglycerol fatty acid ester | 0.05 |
| Sodium chloride | 0.05 |
| Water | 84.598 |
| DDMP | 0.002 |
| Total | 100.00 |

| Alcoholic drink: | |
|---|---|
| (Component) | (Part by weight) |
| 50 vol.% ethanol | 32.0 |
| Sugar | 8.4 |
| Fruit juice | 2.4 |
| DDMP | 0.002 |
| Purified water | 57.198 |
| Total | 100.00 |

### Discussion

Although 2,3-dihydro-3,5-dihydroxy-6-methyl-4H-pyran-4-one obtained from the lactic acid culture supernatant in Example 1 enhanced the kidney sympathetic nervous activity, it caused no rise in blood pressure. Based on these results, it is expected to have a higher safety level relative to hypertension, which brings about a problem as a complication of obesity, than commonly employed compositions having an effect of enhancing sympathetic nervous activity. Since it showed an effect of reducing vagal (parasympathetic) nervous activity of the stomach, it is expected to have an effect of intestinal canal-inhibitively regulating appetite. Moreover, it showed effects of enhancing sympathetic nervous activity of the interscapular brown fat adipose and the epididymal white adipose tissue. Thus, it is expected to have an antiobesity effect through the enhancement of fat burning. The procedures employed in the above Examples 2 and 3 are also advantageous in that the autonomic nervous control functions of different substances can be successively evaluated so long as the test animal survives.

It has been further clarified that 2,3-dihydro-3,5-dihydroxy-6-methyl-4H-pyran-4-one enhances energy metabolism. These results indicate that use of 2,3-dihydro-3,5-dihydroxy-6-methyl-4H-pyran-4-one enables the provision of a medicinal composition, a food or a drink which is efficacious against obesity and/or related complications.

## Claims

1. A medicinal composition, a food or a drink which contains a clinically effective amount of 2,3-dihydro-3,5-dihydroxy-6-methyl-4H-pyran-4-one and a pharmaceutically or nutritionally acceptable additive and has an autonomic nervous control effect.

2. A medicinal composition, food or drink as claimed in claim 1 wherein the autonomic nervous control effect is an effect of enhancing sympathetic nervous activity.

3. A medicinal composition, food or drink as claimed in claim 1 which has an effect of promoting energy metabolism.

4. A medicinal composition, food or drink as claimed in any one of claims 1 to 3 which is to be used for treating a disease or condition relating to autonomic nervous activity or energy metabolism.

5. A medicinal composition, food or drink as claimed in claim 4 wherein the disease or condition relating to autonomic nervous activity or energy metabolism is obesity and/or related complications.

6. A medicinal composition, food or drink as claimed in any one of claims 1 to 5 which contains a microorganism capable of producing 2,3-dihydro-3,5-dihydroxy-6-methyl-4H-pyran-4-one or a processed product thereof.

7. A medicinal composition, food or drink as claimed in any one of claims 1 to 5 which contains a culture supernatant of a microorganism capable of producing 2,3-dihydro-3,5-dihydroxy-6-methyl-4H-pyran-4-one or a processed product thereof.

8. A medicinal composition, food or drink as claimed in claim 6 or 7 wherein the microorganism is a lactic acid bacterium.

9. A medicinal composition, food or drink as claimed in claim 8 wherein the lactic acid bacterium is one belonging to the genus *Streptococcus, Lactobacillus, Leuconostoc, Pediococcus, Bifidobacterium, Tetragenococcus, Weissella, Enterococcus, Melisscoccus, Lactococcus, Carnobacterium, Vagocoacus, Atopobium, Lactosphaera, Oenococcus, Abiotrophia, Paralactobacillus, Granulicatella, Atopobactor, Alkalibacterium* or *Olsenella.*

10. A medicinal composition, food or drink as claimed in claim 6 or 7 wherein the microorganism belongs to *Lactobacillus plantarum* (preferably *Lactobacillus plantarum* SAM 2446 strain (FERM ABP-10438)) or *Lactobacillus brevis* (preferably *Lactobacillus brevis* SAM 2447 strain (FERM ABP-10439)).

11. A method of producing a medicinal composition, food or drink as claimed in any one of claims 1 to 10 which comprises the step of culturing a microorganism capable of producing 2,3-dihydro-3,5-dihydroxy-6-methyl-4H-pyran-4-one in a medium.
